# EUROPÄISCHE PATENTSCHRIFT

(11) **EP 1 479 758 B1**
(45) Veröffentlichungstag und Bekanntmachung des Hinweises auf die Patenterteilung: **25.04.2007**
(21) Anmeldenummer: 04405274.4
(22) Anmeldetag: 30.04.2004
(51) Int. Cl.: B01F 3/04, C12M 1/06, C12M 1/08

(54) **Vorrichtung zum Begasen, vorzugsweise für die mikrobiologische Fermentation und Kultivierung von Zellen**
Device for gassing, particularly for the microbiological fermentation and cultivation of cells
Dispositif pour le gazage, spécialement pour la fermentation microbiologique et la mise en culture de cellules

(30) Priorität: 30.04.2003 CH 7682003
(43) Veröffentlichungstag der Anmeldung: 24.11.2004
(73) Patentinhaber: John Crane UK Limited, Slough, Berkshire SL1 4LU (GB); Kloss, Andreas, 85622 Feldkirchen (DE)
(72) Erfinder: Kloss, Andreas, 85622 Feldkirchen (DE); Koch, Wolfgang, 63110 Rodgau (DE)
(74) Vertreter: Luchs, Willi

(56) Entgegenhaltungen:
- EP-A- 1 473 358
- BE-A- 559 622
- US-A- 3 779 531
- US-B1- 6 250 797

## Beschreibung

Die Erfindung betrifft eine Vorrichtung zum Begasen vorzugsweise für die mikrobiologische Fermentation und Kultivierung von Zellen gemäss dem Oberbegriff des Anspruches 1.

Eine Vorrichtung dieser Art ist beispielsweise aus der EP-B-0 365 621 bekannt. Bei dieser Vorrichtung werden alle in mehreren Ebenen übereinander angeordneten und einen gasdurchlässigen Teil aufweisenden Rührelemente über eine gemeinsame Antriebswelle, die als eine Hohlwelle ausgebildet ist, belüftet. Die Gaszufuhr erfolgt von unten durch die Hohlwelle, von der einzelne in die Rührelemente mündende Belüftungskanäle abzweigen. Bei dieser Vorrichtung ist infolge des hydrostatischen Druckes im Behälter eine gleichmässige Gasverteilung nicht möglich. Das Gas nimmt den Weg des geringsten Widerstandes und tritt vorwiegend in der obersten Belüftungsebene aus.

Im Dokument BE-A-559 622 ist ein Verfahren und eine Vorrichtung zur Dispersion von einem oder mehreren Flüssigkeiten in einer Flüssigkeitsmasse beschrieben. Die Flüssigkeit wird über einen Einlass und jeweils durch eine koaxiale Öffnung eines aus mehreren Rohren bestehenden Rotors und von diesem durch endseitig an diesen Rohren angeordneten tellerförmigen Auslasselemente geführt, wobei letztere am Aussenumfang eine Ringöffnung aufweisen und einen ringförmigen Auslass der Flüssigkeit bilden.

Auch bei einem Verfahren nach der Druckschrift US-A-3,779,531 sind tellerförmige Elemente vorgesehen, welche lochförmige Auslässe aufweisen, die quer zur Rotationsebene der Elemente verlaufen.

Die Druckschrift US-A-6,250,797 zeigt ein Impellersystem, welches aus übereinander angeordneten Rührelementen und in diesen enthaltende Passagen für das Einblasen von Gasen oder Flüssigkeiten zwecks Dispersion dieser eingeblasenen Medien in die mit einer kleineren Viskosität versehenen Flüssigkeit in dem Behälter vorgesehen sind.

Der vorliegenden Erfindung liegt die Aufgabe zugrunde, eine Vorrichtung der eingangs genannten Art zu schaffen, die eine optimale Belüftung des vorzugsweise zur mikrobiologischer Fermentation und Kultivierung von Zellen vorgesehenen Raumes ermöglicht.

Diese Aufgabe wird erfindungsgemäss durch eine Vorrichtung mit den Merkmalen des Anspruches 1 gelöst.

Weitere bevorzugte Ausgestaltungen der erfindungsgemässen Vorrichtung bilden den Gegenstand der abhängigen Ansprüche.

Dadurch, dass für jedes in einer bestimmten Ebene angeordnete Rührelement eine individuelle Gaszufuhr vorgesehen ist, können die einzelnen Belüftungsebenen individuell angesteuert werden, wodurch eine optimale Belüftung bzw. Begasung des Bioreaktors ermöglicht wird. Durch die optimale Belüftung kann die Rührerwirkung auf ein notwendiges Minimum reduziert werden, d.h. die Umdrehungsgeschwindigkeit kann möglichst gering gehalten werden, wodurch eine zellschonende und dennoch eine hohe Produktivität aufweisende Behandlung gewährleistet wird.

Die Erfindung wird nachfolgend anhand der Zeichnung näher erläutert. Es zeigen rein schematisch:
- Fig. 1: ein erstes Ausführungsbeispiel der erfindungsgemässen Vorrichtung in schematischer Seitenansicht;
- Fig. 2: einen Schnitt nach Linie II-II in Fig. 1;
- Fig. 3: ein zweites Ausführungsbeispiel einer erfindungsgemässen Vorrichtung in schematischer Seitenansicht; und
- Fig. 4: Aufsicht der Vorrichtung nach Fig.3.

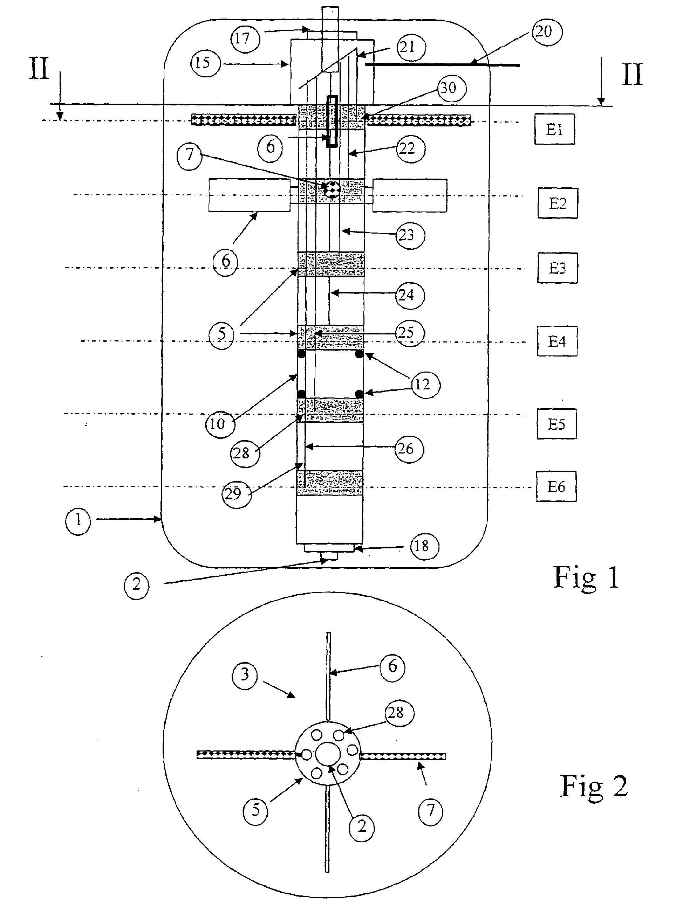

Fig.1 und Fig.2 zeigen einen von einer vertikalen Antriebswelle 2 durchgesetzten Behälter 1, der einen zur mikrobiologischen Fermentation und Kultivierung von Zellen vorgesehenen Bioreaktor bildet. Die Antriebswelle 2 ist mit einem nicht näher dargestellten Antrieb wirkverbunden, wodurch an der Antriebswelle 2 angebrachte Rührelemente 6 in Drehbewegung versetzbar sind. Es sind mehrere, gegebenenfalls sechs Rührelemente 6 in sechs Ebenen E₁ bis E₆ übereinander angeordnet und vorzugsweise über die Behälterhöhe gleichmässig verteilt.

Wie aus Fig. 2 ersichtlich, umfasst jedes Rührelement 3 einen auf der Antriebswelle 2 sitzenden Drehring 5 mit radial wegragenden Rühr- bzw. Begasungselementen 6, 7, bei denen es sich im dargestellten Ausführungsbeispiel um ein Paar von gegenüberliegenden Rührlementen 6 und um ein Paar von diesen gegenüber um 90° versetzten gasdurchlässigen Begasungselementen 7 handelt. Die hohlförmigen Begasungselemente 7 können Membrankerzen oder Elemente mit semipermeablen Membranen sein. Vorzugsweise sind aber auch die Rührelemente 6 hohlförmig ausgestaltet und weisen einen gasdurchlässigen, zum Beispiel mit Belüftungsdüsen versehenen Teil auf (aus der Zeichnung nicht ersichtlich). In Fig. 1 sind einfachheitshalber lediglich die in den obersten zwei Ebenen E1 und E2 angeordneten Rühr- bzw. Begasungselemente 6, 7 dargestellt.

Die Drehringe 5 sind axial durch Distanzringe 10 in einem bestimmten Abstand voneinander, d.h. in den Ebenen E1 bis E6, gehalten. Wie bereits erwähnt, kann es sich um eine gleichmässige Verteilung handeln, oder es kann mit der Wahl entsprechender Distanzringe 10 eine andere, den spezifischen Bedürfnissen angepasste Anordnung realisiert werden. Zwischen jedem Drehring 5 und den benachbarten Distanzringen 10 ist jeweils ein Dichtungsring 12 vorhanden.

Im oberen Bereich des Behälters 1 ist auf dem obersten Drehring 5 eine Verteilereinrichtung 15 mit einem dazwischen angeordneten Dichtungsring 12 aufgesetzt. Diese Verteilereinrichtung 15 wird zusammen mit den Drehringen 5 und den Distanzringen 10 mittels einer Spannvorrichtung 17, 18 auf der Antriebswelle 2 axial gehalten.

Die an sich bekannte Verteilereinrichtung 15 ist dazu bestimmt, das dem Bioreaktor zuzuführende Gas, z.B. Sterilluft, reinen Sauerstoff oder ein Sauerstoff/Stickstoff-Gemisch, aus einer Hauptzufuhrleitung 20 in eine Anzahl von vertikalen Zufuhrkanälen 21 bis 26 zuzuführen, durch welche es erfindungsgemäss individuell zu jedem in einer bestimmten Ebene E1 bis E6 angeordneten Rührelement 3 geleitet wird. Die Anzahl der Zufuhrkanäle 21 bis 26 entspricht somit der Anzahl der Rührelemente 3 bzw. der Anzahl der Ebenen E1 bis E6. Die Hauptzufuhrleitung 20 ihrerseits besteht vorteilhaft ebenfalls aus einer Anzahl von einzelnen Leitungen (nicht dargestellt), die mit jeweils mindestens einem Zufuhrkanal 21 bis 26 verbunden sind, so dass in jeder Ebene E1 bis E6 eine individuelle Gaszufuhr ermöglicht ist. Im Prinzip könnten aber beispielsweise in zwei benachbarten Ebenen die gleiche Menge Gas unter dem gleichen Druck zugeführt werden. Diese beiden Ebenen könnten dann an die gleiche Gasquelle angeschlossen sein. Theoretisch könnten in der Verteilereinrichtung entsprechende Ventile angeordnet sein, welche die Gasmenge und/oder den Gasdruck von der einen Hauptzufuhrleitung 20 zu den Zufuhrkanälen 21 bis 26 steuern würden.

Die an die Verteilereinrichtung 15 anschliessenden Zufuhrkanäle 21 bis 26 sind durch in Längsrichtung verlaufende Bohrungen 28 in den Drehringen 5 bzw. durch in Längsrichtung verlaufende Bohrungen 29 in den Distanzringen 10 gebildet. Der oberste Drehring 5 ist mit einer der Gesamtzahl der Begasungselemente 6 entsprechenden Anzahl von Bohrungen 28, im gegebenen Fall mit deren sechs, ausgestattet, jeder weitere darunterliegende Drehring 5 weist eine Bohrung 28 weniger auf, bis schliesslich der unterste Drehring 5 mit lediglich einer Bohrung 28 versehen ist.

Die bereits erwähnten Begasungselemente 7 sind in radiale Bohrungen im jeweiligen Drehring 5 eingesetzt, wobei sich diese Bohrungen bis zu dem für die Gaszufuhr zum betreffenden Begasungselemente 7 zuständigen vertikalen Zufuhrkanal 21 bis 26 erstrecken, in diesen münden und jeweils eine Querverbindung 30 zum entsprechenden Element 6, 7 bilden. So sind zum Beispiel die Belüftungskanäle 30 des obersten Begasungselementes 7 an den Zufuhrkanal 21, die des darunterliegenden Begasungselement 7 an den Zufuhrkanal 22 usw., bis zum untersten Begasungselement 7 und dem Zufuhrkanal 26, angeschlossen.

Dies bedeutet, dass die einzelnen Belüftungsebenen E1 bis E6 individuell angesteuert werden können, wodurch eine optimale Belüftung bzw. Begasung des Bioreaktors ermöglicht wird. Durch die optimale Belüftung kann die Rührerwirkung auf ein notwendiges Minimum reduziert werden, d.h. die Umdrehungsgeschwindigkeit kann möglichst gering gehalten werden, wodurch eine zellschonende und dennoch eine hohe Produktivität aufweisende Behandlung gewährleistet wird. Die Rührerwirkung ist nicht mehr auf das Herumwirbeln des Gasstroms und die mechanische Zerstörung der grossen Gasblasen ausgerichtet, sondern hat vorwiegend die Aufgabe, der Absatztendenz des Produktbildners entgegen zu wirken.

Die Gaszuführung erfolgt in getrennten, einzeln regulierbaren Zuleitungen zur Verteilereinrichtung 15, in welcher unter strenger Beachtung der steriltechnischen und mechanischen Anforderungen die statische Zuführung in die drehende Welle 2 transformiert werden. In der Welle erfolgt in getrennter Kanalzuführung die Verteilung des Gases zur gewünschten Ebene. Dort tritt das Gas jeweils in die seitlich angebrachten Rührflügel 6 bzw. Membrankerzen ein und wird in drehender Bewegung in den Bioreaktor eingeleitet.

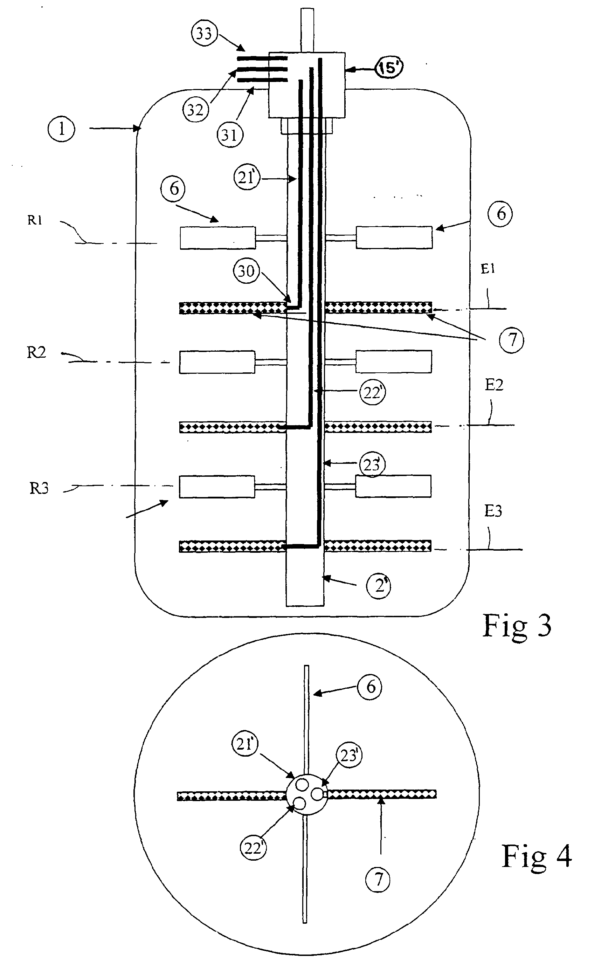

Fig. 3 und 4 zeigen eine Ausführungsvariante einer Vorrichtung, bei welcher alternierend Belüftungsebenen E1 bis E3 und Rührebenen R1 bis R3 vorgesehen sind. Bei den Belüftungsebenen E1 bis E3 sind längliche Begasungselemente 7, indessen bei den Rührebenen R1 bis R3 Rührelemente 6 vorgesehen sind. Diese Begasungs- bzw. Rührelemente sind an der drehbaren Welle 2' befestigt, wobei pro Ebene vorzugsweise zwei oder vier solcher Elemente von der Welle wegragend angeordnet sind. Die in die Begasungselemente 7 führenden, vertikalen Zufuhrkanäle 21' bis 23' und Querverbindungen 30' sind hierbei direkt in/an der Antriebswelle 2' angefertigt, welche für die Belüftung bzw. Begasung dieser Begasungselemente 7 dienen. Die Zufuhrkanäle könnten aber auch durch entsprechende Rohrleitungen realisiert sein.

Die Verteilereinrichtung 15' ist wiederum im oberen Bereich des Behälters 1 untergebracht, vorzugsweise in der Behälterwandung. Es wäre allerdings durchaus möglich, diese auch ausserhalb des Behälters anzuordnen, wobei jedoch die dargestellte Variante konstruktiv einfacher ist.

Diese Verteilereinrichtung 15' besteht einerseits aus einer Dichtungsanordnung und andererseits ist die Verteilung der individuellen Luft- bzw. Gaszufuhr in die in der Welle 2' enthaltenden vertikalen Zufuhrkanäle 21' bis 23' mittels den horizontalen stationären Leitungen 31 bis 33 verwirklicht, was nur schematisch angedeutet ist.

Die optimale Auslegung des Bioreaktors ist immer produktabhängig. Die erfindungsgemässe Vorrichtung erlaubt, die Menge des benötigten Gases sowie die notwendige Umdrehungsgeschwindigkeit der Rührelemente 3 dem jeweiligen Verfahren in einfacher Weise anzupassen.

Anstelle der Begasung der Rührelemente von der Antriebswelle aus könnten sie auch von aussen ähnlich wie dies in der EP-A-0 365 621 veranschaulicht ist, begast werden, wobei wiederum erfindungsgemäss die Begasung der einzelnen Rührelemente individuell in der Menge, dem Druck und/oder mit unterschiedlichen Gasen erfolgen kann.

In einer Ebene (E1, ...) sind vorteilhaft zwei oder mehr Rührelemente (3) vorgesehen. Im Prinzip kann aber auch nur eines pro Ebene installiert sein. Unter den zu behandelnden Zellen sind im weitesten Sinne auch andere Organismen, wie Mikroorganismen oder ähnlichem zu verstehen.

Für die Belüftung eignen sich verschiedene Medien, insbesondere Gase, Luft oder Gas/Luftgemische.

## Patentansprüche

1. Vorrichtung zum Begasen vorzugsweise für die mikrobiologische Fermentation und Kultivierung von Zellen in einem geschlossenen Raum, mit einem sterilisierbaren Behälter (1), einer im Behälter (1) angeordneten Antriebswelle (2; 2'), mit einer Anzahl von an der Antriebswelle (2; 2') angebrachten Begasungselementen (7) und Rührelementen (6), welche in mehreren Ebenen (E1; E2; E3; E4; E5; E6) übereinander angeordnet sind, wobei die Begasungselemente (7) mindestens einen gasdurchlässigen Teil aufweisen und jeweils mit einer individuellen Gaszufuhr versehen sind, wobei für die individuelle Gaszufuhr eine Verteilereinrichtung (15; 15') vorhanden ist, **dadurch gekennzeichnet, dass** die länglich ausgebildeten Begasungselemente (7) gleichmässig über die Behälterhöhe verteilt angeordnet sind, wobei die Verteilereinrichtung (15; 15') mit zu vertikalen Gaszufuhrkanälen (21', 22', 23') führenden horizontalen stationären Leitungen (31, 32, 33) versehen ist, wobei diese zu den einzelnen Ebenen (E1; E2; E3) führenden vertikalen Gaszufuhrkanäle (21', 22', 23') direkt in der Antriebswelle (2') oder durch Rohrleitungen angefertigt sind.

2. Vorrichtung nach Anspruch 1, **dadurch gekennzeichnet, dass** das Gas von einer Gasdruckquelle über eine Verteilereinrichtung (15; 15') und je einen vertikalen, bis zu der jeweiligen Ebene (E1; E2; E3; E4; E5; E6) führenden Zuführkanal (21, 22, 23, 24, 25, 26; 21', 22', 23') und den an diesen anschliessenden und jeweils zu dem gasdurchlässigen Teil des entsprechenden Begasungs- bzw. Rührelementes (7, 6) führenden Querverbindung (30; 30') zugeführt wird.

3. Vorrichtung nach Anspruch 2, **dadurch gekennzeichnet, dass** die zu den einzelnen Ebenen (E1; E2; E3) führenden vertikalen Gaszufuhrkanäle (21', 22', 23') in der Antriebswelle (2') angefertigt sind, wobei die Antriebswelle (2') in jeder Ebene (E1; E2; E3) die Belüftungskanäle (30') bildende Querverbindungen aufweist, in welche hohlförmige Rühr- bzw. Begasungselemente (6, 7) des jeweiligen Rührelementes (3) einsetzbar sind, die den gasdurchlässigen Teil bilden oder aufweisen.

4. Vorrichtung nach einem der Ansprüche 1 bis 3, **dadurch gekennzeichnet, dass** die Anzahl und Anordnung der Begasungsebenen und der Rühr- bzw. Begasungselemente (3) veränderbar ist.

5. Vorrichtung nach einem der Ansprüche 2 bis 4, **dadurch gekennzeichnet, dass** die Verteilereinrichtung (15; 15') im oberen Bereich des Behälters (1) oder ausserhalb des Behälters (1) angeordnet ist.

6. Vorrichtung nach einem der Ansprüche 3 bis 5, **dadurch gekennzeichnet, dass** die Begasungselemente (7) als Membranelemente gebildet sind.

7. Vorrichtung nach einem der Ansprüche 1 bis 6, **dadurch gekennzeichnet, dass** die Begasung der einzelnen Rührelemente individuell in der Menge, im Druck und/oder mit unterschiedlichen Gasen durchführbar ist.

8. Vorrichtung nach einem der Ansprüche 1 bis 7, **dadurch gekennzeichnet, dass** in der Verteilereinrichtung die Gasverteilungsfunktion mit der Wellendichtungsfunktion in einer mechanischen Einheit realisiert ist.

9. Vorrichtung nach einem der Ansprüche 1 bis 7, **dadurch gekennzeichnet, dass** in der Verteilereinrichtung (15) die Gasverteilungsfunktion mit der Wellendichtungsfunktion in einer mechanischen Einheit realisiert ist, wobei diese Verteilereinrichtung (15) beim Eintritt bzw. am Austritt der Antriebswelle (2, 2') im Behälter (1) angeordnet ist, wobei dies am oberen bzw. am unteren Ende des Behälters oder seitlich am Behälter (1) sein kann.

10. Vorrichtung nach einem der Ansprüche 1 bis 7, **dadurch gekennzeichnet, dass** die Dichtungsfunktion der Verteilereinrichtung mechanisch und/oder dass die mechanischen Dichtungskomponenten auch als Gasverteilungskomponenten vorgesehen sind.

11. Vorrichtung nach einem der Ansprüche 1 bis 7, **dadurch gekennzeichnet, dass** das Dichtmedium der mechanischen Dichtung ein Gas ist, welches gleichsam von dort durch die Antriebswelle (2, 2') zum Begasungs- bzw. Rührelement geführt ist.

## Claims

1. Means for gassing, preferably for the microbiological fermentation and cultivation of cells, within an enclosed space, with a sterilisable container (1), a drive shaft (2; 2') located within the container (1) with a multitude of gassing elements (7) affixed to the drive shaft (2; 2'), and stirrer elements (6) located on several levels (E1; E2; E3; E4; E5; E6) above each other, whereby the gassing elements (7) comprise at least one gas permeable part and are each equipped with an individual gas supply, whereby a distribution means (15; 15') is provided for the individual gas supply, **characterised in that** the longitudinally formed gassing elements (7) are evenly distributed across the container height, whereby the distribution means (15; 15') is equipped with stationary horizontal lines (31, 32, 33) leading to vertical gas supply channels (21', 22', 23'), whereby the same are manufactured as vertical gas supply channels (21', 22', 23') leading directly to the individual levels (E1; E2; E3) within the drive shaft (2') or by means of pipes.

2. Means according to Claim 1, **characterised in that** the gas is supplied from a gas pressure source via a distribution means (15; 15') and a vertical supply channel (21, 22, 23, 24, 25, 26; 21', 22', 23') leading to the relevant level (E1; E2; E3; E4; E5; E6) each, and then to the cross connection (30; 30') adjacent to the former leading to the relevant gas permeable part of the relevant gassing, e.g. stirrer element (7, 6).

3. Means according to Claim 2, **characterised in that** the vertical gas supply channels (21', 22', 23') leading to the individual levels (E1; E2; E3) are provided in the drive shaft (2'), whereby the drive shaft (2') comprises the cross connections forming the ventilation channels (30') on every level (E1; E2; E3), in which hollow stirrer, e.g. gassing elements (6, 7) of the relevant stirrer element (3) can be used, forming or comprising the gas permeable part.

4. Means according to one of the Claims 1 to 3, **characterised in that** the number and the arrangement of the gassing levels and the stirrer, e.g. gassing elements (3) are changeable.

5. Means according to one of the Claims 2 to 4, **characterised in that** the distribution means (15; 15') is located in the upper area of the container (1) or outside of the container (1).

6. Means according to one of the Claims 3 to 5, **characterised in that** the gassing elements (7) take the form of membrane elements.

7. Means according to one of the Claims 1 to 6, **characterised in that** the gassing of the individual stirrer elements can be carried out individually with varying quantity, pressure and/or with different gases.

8. Means according to one of the Claims 1 to 7, **characterised in that** the gas distribution function is realised by means of the shaft sealing function within a mechanical unit in the distribution means.

9. Means according to one of the Claims 1 to 7, **characterised in that** the gas distribution function in the distribution means (15) is realised by means of the shaft sealing function in a mechanical unit, whereby this distribution means (15) is located at the inlet, e.g. at the outlet of the drive shaft (2, 2') in the container (1), whereby the same can be located at the upper, e.g. at the lower end of the container or at the side of the container (1).

10. Means according to one of the Claims 1 to 7, **characterised in that** the sealing function of the distribution means is envisaged mechanically and/or **in that** the mechanical sealing components are also envisaged as gas distribution components.

11. Means according to one of the Claims 1 to 7, **characterised in that** the sealing medium of the mechanical seal is a gas, which is supplied from there through the drive shaft (2' 2') to the gassing, e.g. stirrer element.

## Revendications

1. Dispositif d'envoi de gaz, de préférence pour la fermentation microbiologique et la mise en culture de cellules dans un espace fermé, comprenant un récipient (1) pouvant être stérilisé, un arbre (2, 2') d'entraînement qui est disposé dans le récipient (1) et qui a un certain nombre d'éléments (7) d'envoi de gaz et d'éléments (6) d'agitation, qui sont montés sur l'arbre (2, 2') d'entraînement qui sont superposés dans plusieurs plans (E1, E2, E3, E4, E5, E6), les éléments (7) d'envoi de gaz ayant au moins une partie perméable au gaz et étant munis respectivement d'un apport individuel de gaz, un dispositif (15, 15') répartiteur étant présent pour l'apport individuel de gaz, **caractérisé en ce que** les éléments (7) d'envoi de gaz, de constitution oblongue, sont répartis uniformément sur la hauteur du récipient, le dispositif (15, 15') répartiteur étant muni de conduits (31, 32, 33) horizontaux et fixes menant aux canaux (21', 22', 23') verticaux d'apport de gaz, ces canaux (21', 22', 23') verticaux menant aux divers plans (E1, E2, E3) étant ménagés directement dans l'arbre (2') d'entraînement ou par des conduits tubulaires.

2. Dispositif suivant la revendication 1, **caractérisé en ce que** le gaz est apporté d'une source de gaz comprimé par un dispositif (15, 15') répartiteur et est envoyé respectivement à un canal (21, 22, 23, 24, 25, 26 ; 21', 22', 23') d'apport menant au plan (E1, E2, E3, E4, E5, E6) respectif, et à la liaison (30, 30') transversale se raccordant à celui-ci et menant respectivement à la partie perméable au gaz de l'élément (7, 6) d'envoi de gaz ou d'agitation correspondant.

3. Dispositif suivant la revendication 2, **caractérisé en ce que** les canaux (21', 22', 23') verticaux d'apport de gaz menant aux divers plans (E1, E2, E3) sont ménagés dans l'arbre (2') d'entraînement, l'arbre (2') d'entraînement ayant dans chaque plan (E1, E2, E3) les liaisons transversales formant les canaux (30') d'aération, liaisons dans lesquelles des éléments (6, 7) creux d'agitation ou d'envoi de gaz de l'élément (3) respectif d'agitation peuvent être insérés et forment la partie perméable au gaz ou la comporte.

4. Dispositif suivant l'une des revendications 1 à 3, **caractérisé en ce que** le nombre et la position des plans d'envoi de gaz et des éléments (3) d'agitation d'envoi de gaz peuvent être modifiés.

5. Dispositif suivant l'une des revendications 2 à 4, **caractérisé en ce que** le dispositif (15, 15') répartiteur est disposé dans la partie supérieure du récipient (1) ou à l'extérieur du récipient (1).

6. Dispositif suivant l'une des revendications 3 à 5, **caractérisé en ce que** les éléments (7) d'envoi de gaz sont constitués sous la forme d'éléments à membrane.

7. Dispositif suivant l'une des revendications 1 à 6, **caractérisé en ce que** l'envoi de gaz aux divers éléments d'agitation peut s'effectuer individuellement en quantité, en pression et/ou avec des gaz différents.

8. Dispositif suivant l'une des revendications 1 à 7, **caractérisé en ce que** la fonction de répartition du gaz est réalisée dans le dispositif répartiteur avec la fonction d'étanchéité par ondulation en une unité mécanique.

9. Dispositif suivant l'une des revendications 1 à 7, **caractérisé en ce que** la fonction de répartition du gaz est réalisée dans le dispositif (15) répartiteur avec la fonction d'étanchéité par ondulation dans une unité mécanique, ce dispositif (15) répartiteur étant disposé à l'entrée ou à la sortie de l'arbre (2, 2') d'entraînement dans le récipient (1), cela pouvant se faire à l'extrémité supérieure ou à l'extrémité inférieure du récipient ou latéralement au récipient (1).

10. Dispositif suivant l'une des revendications 1 à 7, **caractérisé en ce que** la fonction d'étanchéité du dispositif répartiteur est prévue mécaniquement et/ou **en ce que** les éléments mécaniques d'étanchéité sont prévus aussi sous la forme d'éléments de répartition du gaz.

11. Dispositif suivant l'une des revendications 1 à 7, **caractérisé en ce que** le milieu d'étanchéité de l'étanchéité mécanique est un gaz qui est guidé de la même façon par à l'élément d'envoi de gaz ou d'agitation en passant par l'arbre (2, 2') d'entraînement.
